# EUROPEAN PATENT APPLICATION

(11) **EP 4 260 856 A1**
(43) Date of publication of application: **18.10.2023**
(21) Application number: 21902368.6
(22) Date of filing: 19.11.2021
(51) Int. Cl.: A61K 31/401, A61K 31/19, C01D 15/00, A61P 25/00, C07D 207/16, C07C 53/124, C07C 51/43

(54) **ORGANIC ACID LITHIUM AMINO ACID SALT, CRYSTAL FORM, COMPOSITION, AND APPLICATION**

(30) Priority: 09.12.2020 CN 202011424798
(71) Applicant: Anyu Biotechnology (Hangzhou) Co., Ltd., Hangzhou, Zhejiang 310000 (CN)
(72) Inventor: TAN, Jun, Hangzhou, Zhejiang 310000 (CN); CHEN, Binhui, Hangzhou Medical Town, Weiken Road, Xiasha Street, Qiantang New District Hangzhou, Zhejiang 310000 (CN); LI, Song, Dalian, Liaoning 310000 (CN); TANG, Qinmin, Anshun, Guizhou 561116 (CN); CHEN, Jiang, Guiyang, Guizhou 550004 (CN)
(74) Representative: Zaboliene, Reda
(86) International application number: PCT/CN2021/131789
(87) International publication number: WO 2022/121661

(57) **Abstract**

The present disclosure discloses a lithium organic acid-amino acid salt. A lithium organic acid is one or more of lithium isobutyrate, lithium n-butyrate, lithium lactate, lithium citrate or lithium cholesterol; an amino acid is one of L-proline, valine, lysine or artificially synthetic amino acids; and the lithium organic acid-amino acid salt is a salt formed by the lithium organic acid and the amino acid. The present disclosure further discloses a crystal form, a preparation method and an application of the salt. The lithium organic acid-amino acid salt of the present disclosure has a positive curative effect and a preventive effect on recurrent attacks of mania and depression in bipolar disorder, and can delay degenerative diseases of the central nervous system to realize better distribution in the central nervous system, so that while the efficacy for diseases of the central nervous system is improved, the clinical dosage of the lithium salt is reduced, and the occurrence of peripheral adverse reactions is avoided.

## Description

### FIELD OF TECHNOLOGY

The present disclosure belongs to the field of medicine, and specifically relates to a novel lithium organic acid-amino acid salt, and a crystal form, a composition, a preparation process and an application thereof in the field of nervous and mental diseases.

### BACKGROUND

A lithium salt can be used in the field of mental diseases, has a good curative effect and a preventive effect on recurrent attacks of mania and depression in bipolar disorder, and also has a unique prevention effect on a suicide risk. Recent studies have also found that the lithium salt can play a neuroprotective role by acting on GSK-3, WNT, AKT and neurotransmitters. The lithium salt has selective activity for GSK-3β, and mainly has two action ways for inhibiting the GSK-3β: (1) directly inhibiting the activity of the GSK-3β as a competitive inhibitor of Mg²⁺; and (2) indirectly inhibiting the activity of the GSK-3β by increasing the expression of phosphorylated AKT, phosphorylated GSK-3β (ser9) and MCL-1. Such protective role has the potential for prevention and treatment of neurodegenerative diseases, including Alzheimer's disease. At present, lithium salts commonly used in clinical applications include lithium carbonate, lithium citrate, lithium acetate and long-acting lithium salts, among which the lithium carbonate is most commonly used.

At present, inorganic lithium salts commonly used in clinical applications have a narrow safety range, and long-term medication is usually required in the treatment of mental diseases. Due to the long-term medication, potential kidney damage and thyroid dysfunction are caused, and as a result, clinical applications of medicines are greatly limited. Meanwhile, after long-term use of lithium inorganic acid salts (such as lithium chloride and lithium carbonate) commonly used in clinical applications at present, disorder of the pH value of the blood will also be caused, leading to metabolic acidosis and aggravated burden of the kidney. In recent years, it has been found that a variety of small molecule organic acids, including butyric acid, isobutyric acid, valproic acid and folic acid, have an important influence on the central nervous system, which can effectively relieve anxiety, depression and other emotional abnormalities and delay degenerative diseases of the central nervous system. In addition, organic acid salts usually have great differences from inorganic acid salts in absorption, distribution and metabolism characteristics, so that in vivo distribution defects of existing inorganic lithium salts are expected to be changed. Therefore, lithium organic acid salts have a higher value of development of new medicines and a better market prospect.

### SUMMARY

Technical problems to be solved: A purpose of the present disclosure is to provide a novel lithium organic acid-amino acid salt, so as to solve the problems of insufficient efficacy and large toxicity and side effects of existing lithium preparations.

Meanwhile, the present disclosure provides a crystal form of a lithium organic acid-L-proline salt and a composition.

The present disclosure also provides a preparation method of the lithium organic acid-amino acid salt, the crystal form or the composition.

The present disclosure further provides an application of the lithium organic acid-amino acid salt, the crystal form or the composition in preparation of a medicine for treatment of a nervous disease or a medicine for treatment of a mental disease.

A lithium organic acid-amino acid salt is provided, where a lithium organic acid is one or more of lithium isobutyrate, lithium n-butyrate, lithium lactate, lithium citrate or lithium cholesterol and other lithium organic acids; an amino acid is one of L-proline, valine, lysine or other natural amino acids or artificially synthetic amino acids; and the lithium organic acid-amino acid salt is a salt formed by the lithium organic acid and the amino acid.

As a preference, the lithium organic acid is lithium isobutyrate.

As a preference, the amino acid is L-proline.

As a preference, the lithium organic acid-amino acid salt is a lithium isobutyrate-L-proline salt.

As a preference, the lithium isobutyrate and the amino acid in the lithium organic acid-amino acid salt are combined at a molar ratio of (0.8-1.2):(0.8-1.2). As a further preference, the lithium organic acid-amino acid salt is a lithium isobutyrate-L-proline salt. The lithium isobutyrate and the L-proline in the lithium organic acid-amino acid salt are combined at a molar ratio of (0.8-1.2):(0.8-1.2).

As a preference, the lithium isobutyrate and the L-proline in the lithium organic acid-amino acid salt are combined at a molar ratio of 1:1. As a further preference, the lithium organic acid-amino acid salt is a lithium isobutyrate-L-proline salt. The lithium isobutyrate and the L-proline in the lithium organic acid-amino acid salt are combined at a molar ratio of 1:1.

A crystal form of a lithium organic acid-amino acid salt is provided, where lithium isobutyrate and L-proline in the lithium organic acid-amino acid salt are combined at a molar ratio of 1:1, and the crystal form has characteristic peaks at a diffraction angle (2θ) of 8.201°±0.2°, 11.735°±0.2°, 20.395°±0.2°, 23.669°±0.2° and 24.930°±0.2° in an X-ray powder diffraction pattern.

A crystal form of a lithium organic acid-amino acid salt is provided, where lithium isobutyrate and L-proline in the lithium organic acid-amino acid salt are combined at a molar ratio of 1:1, and the crystal form has characteristic peaks at a diffraction angle (2θ) of 7.909°±0.2°, 8.201°±0.2°, 11.735°±0.2°, 16.525°±0.2°, 20.395°±0.2°, 23.669°±0.2°, 24.930°±0.2°, 30.232°±0.2°, 31.427°±0.2° and 33.451°±0.2° in an X-ray powder diffraction pattern.

A crystal form of a lithium organic acid-amino acid salt is provided, where lithium isobutyrate and L-proline in the lithium organic acid-amino acid salt are combined at a molar ratio of 1:1, and the crystal form has characteristic peaks at a diffraction angle (2θ) of 8.255°±0.2°, 11.782°±0.2°, 16.584°±0.2°, 16.811°±0.2°, 18.711°±0.2°, 18.800°±0.2°, 20.451°±0.2°, 23.715°±0.2°, 25.017°±0.2°, 25.040°±0.2°, 30.281°±0.2°, 31.490°±0.2°, 33.537° ±0.2° and 35.886°±0.2° in an X-ray powder diffraction pattern.

Meanwhile, the present disclosure provides a preparation method of the lithium organic acid-amino acid salt (preferably lithium isobutyrate-L-proline salt) described in any one of the technical solutions or the crystal form described in any one of the technical solutions. The preparation method includes: preparation by a single solvent method or a mixed solvent method.

The preparation method of the lithium organic acid-amino acid salt (preferably lithium isobutyrate-L-proline salt) described in any one of the technical solutions, or the preparation method of the crystal form described in any one of the technical solutions includes:
in the single solvent method, adding an appropriate amount of a solvent to the lithium organic acid and the amino acid, conducting heating reflux for 1-5 hours, and conducting heat filtration, followed by natural cooling and crystallization;
or in the mixed solvent crystallization method, adding an appropriate amount of a good solvent to the lithium organic acid and the amino acid, conducting heating reflux for dissolution, then adding an appropriate amount of a poor solvent for precipitation of a solid, supplementing the good solvent for redissolution of the solid, and conducting reflux for 1-5 hours, followed by natural cooling and crystallization.

As a preference, in the single solvent method, the solvent is n-butanol; and in the mixed solvent crystallization method, the good solvent is ethanol, and the poor solvent is tetrahydrofuran.

In the single solvent method, the added amount of the solvent is determined based on the situation that proper dissolution is realized under the heating reflux; and in the mixed solvent crystallization method, the good solvent is added first to realize proper dissolution, then the poor solvent is added to realize proper precipitation, the good solvent is supplemented to realize dissolution by heating, and the reflux is conducted for 1-5 hours, followed by natural cooling and crystallization.

A crystal form of a lithium organic acid-amino acid salt is provided, where lithium isobutyrate and L-proline in the lithium organic acid-amino acid salt are combined at a molar ratio of 1:1, and the crystal form has characteristic peaks at a diffraction angle (2θ) of 7.909°, 8.201°, 11.735°, 16.525°, 20.395°, 23.669°, 24.930°, 30.232°, 31.427° and 33.451° in an X-ray powder diffraction pattern.

A preparation method of the crystal form includes: adding an appropriate amount of a good solvent (n-butanol) to equal chemical equivalents of the lithium isobutyrate and the L-proline, conducting heating reflux to realize proper dissolution, and recording the use amount of the solvent; conducting stirring for about 3 hours, and conducting heat filtration, followed by natural cooling and crystallization; and filtering a solid, and conducting vacuum drying until a constant weight to obtain the crystal form of a lithium organic acid-amino acid salt.

A crystal form of a lithium organic acid-amino acid salt is provided, where lithium isobutyrate and L-proline in the lithium organic acid-amino acid salt are combined at a molar ratio of 1:1, and the crystal form has characteristic peaks at a diffraction angle (2θ) of 8.255°, 11.782°, 16.584°, 16.811°, 18.711°, 18.800°, 20.451°, 23.715°, 25.017°, 25.040°, 30.281°, 31.490°, 33.537° and 35.886° in an X-ray powder diffraction pattern.

A preparation method of the crystal form includes: adding an appropriate amount of a good solvent to equal chemical equivalents of the lithium isobutyrate and the L-proline, conducting heating reflux to realize proper dissolution, and then adding an appropriate amount of a poor solvent until proper precipitation of a solid; supplementing the good solvent to realize redissolution of the solid, and conducting reflux for 3 hours, followed by natural cooling and crystallization; and filtering a solid, and conducting vacuum drying until a constant weight to obtain a white solid of the crystal form.

A pharmaceutical composition is provided, where the pharmaceutical composition includes the lithium organic acid-amino acid salt described in any one of the technical solutions or the crystal form described in any one of the technical solutions.

The present disclosure provides an application of the lithium organic acid-amino acid salt described in any one of the technical solutions, the crystal form described in any one of the technical solutions, or the pharmaceutical composition in preparation of a medicine for treatment of a disease that benefits from the inhibition of the activity of GSK-3β, the increase of the expression of a β-catenine protein, or the inhibition of the expression of a tau protein.

As a preference, the disease includes one or more of a neurodegenerative disease and a mental disease.

As a preference, the neurodegenerative disease is one or more of Alzheimer's disease, Parkinson's disease, Huntington's disease, epilepsy, amyotrophic lateral sclerosis and spinocerebellar ataxia.

As a preference, the mental disease is one or more of mild, moderate, or severe depression and bipolar disorder.

The novel lithium isobutyrate-L-proline salt is prepared by the applicant based on the single solvent method and the mixed solvent method with the lithium isobutyrate and the L-proline as raw materials. In the single solvent method, an appropriate amount of a good solvent is added to different chemical equivalents of the lithium isobutyrate and the L-proline, heating reflux is conducted for 1-5 hours, and heat filtration is conducted, followed by natural cooling and crystallization. In the mixed solvent crystallization method, an appropriate amount of a good solvent is added to different chemical equivalents of the lithium isobutyrate and the L-proline, heating reflux is conducted for dissolution, then an appropriate amount of a poor solvent is added for precipitation of a solid, the good solvent is supplemented for redissolution of the solid, and reflux is conducted for 1-5 hours, followed by natural cooling and crystallization. According to XRD and hydrogen spectrum verification, a novel salt is formed by the lithium isobutyrate and the L-proline. On this basis, the potential application value and safety of the salt for the treatment of Antialzheimer's disease are preliminarily evaluated.

According to the present disclosure, the nuclear magnetic hydrogen spectrum of the obtained lithium isobutyrate-L-proline salt, lithium isobutyrate and L-proline is separately detected under same conditions, and then superimposed. According to a superimposed pattern, it can be seen that the hydrogen spectrum of the solid precipitated under the condition of a single solvent or the solid obtained after mixed crystallization is different from that of the single compound lithium isobutyrate, that of the single compound L-proline or the superimposed spectrum of the two single compounds. Therefore, it can be determined that the solid precipitated under the condition of n-butanol is a salt different from the two raw materials.

According to the present disclosure, the lengths of C-O bonds of the L-proline in a single crystal lattice are measured by Mercury software, which are 1.233 Å and 1.268 Å respectively, and the ratio is 1.028. The lengths of the C-O bonds of the L-proline are basically the same, indicating that due to obvious proton transfer of the L-proline in a complex, the lengths of the C-O bonds of a carboxylate group tend to be the same, and it is determined that a prepared product is a lithium isobutyrate-L-proline salt.

Experimental results show that the lithium isobutyrate-L-proline salt significantly inhibits the activity of GSK-3β, showing that the expression of phosphorylated GSK-3β (Ser9) is significantly increased in a dose-dependent manner, and the effect is significantly better than that of an existing lithium preparation, namely lithium carbonate, commonly used in clinical applications. Meanwhile, the lithium isobutyrate-L-proline salt significantly increases the expression of a β-catenine protein, indicating that the salt has the effect of promoting proliferation and survival of cells, and the effect is significantly better than that of the lithium carbonate. An SH-SY5Y cell experiment shows that the lithium isobutyrate-L-proline salt can significantly down-regulate the expression of a neurodegeneration related protein (tau), indicating that the salt has the effect of inhibiting degeneration of nerve cells, and the effect is significantly better than that of the lithium carbonate. Moreover, compared with the lithium carbonate, the cytotoxicity of the lithium isobutyrate-L-proline salt is significantly reduced, and the expression of Notch is not affected while the GSK-3β is inhibited. The lithium isobutyrate-L-proline salt has no significant influence on the survival of HEK293 cells. Finally, experimental results further prove that compared with the lithium carbonate, the lithium isobutyrate-L-proline salt has a higher blood lithium level, while having no obvious difference in the level of lithium in the brain.

The present disclosure has the following beneficial effects. The present disclosure has a positive curative effect and a preventive effect on recurrent attacks of mania and depression in bipolar disorder, and also has a unique prevention effect on a suicide risk. Meanwhile, the salt can effectively relieve anxiety, depression and other emotional abnormalities and delay degenerative diseases of the central nervous system. Moreover, organic acid salts usually have great differences from inorganic acid salts in absorption, distribution and metabolism characteristics, so that in vivo distribution defects of existing inorganic lithium salts are changed. In addition, the novel small molecule organic lithium salt designed and developed by the present disclosure has better safety while the efficacy for diseases of the central nervous system is improved, so that the clinical dosage of the lithium salt is reduced, and the occurrence of peripheral adverse reactions is avoided.

### BRIEF DESCRIPTION OF THE DRAWINGS

FIG. 1 shows the hydrogen spectrum of a single compound lithium isobutyrate;
FIG. 2 shows the hydrogen spectrum of a single compound L-proline;
FIG. 3 shows the hydrogen spectrum of a physical mixture of equal chemical equivalents of lithium isobutyrate and L-proline;
FIG. 4 shows the hydrogen spectrum of a solid precipitated under the condition of n-butanol;
FIG. 5 is an XRD pattern of the solid precipitated under the condition of n-butanol;
FIG. 6 is an XRD pattern of the single compound L-proline;
FIG. 7 is an XRD pattern of the single compound lithium isobutyrate;
FIG. 8 is a superimposed XRD pattern;
FIG. 9 shows the hydrogen spectrum of a solid precipitated in a mixed solvent of ethanol and tetrahydrofuran;
FIG. 10 is an XRD pattern of the solid precipitated in the mixed solvent of ethanol and tetrahydrofuran;
FIG. 11 is an X-ray single crystal diffraction pattern of a lithium isobutyrate-L-proline complex;
FIG. 12 shows experimental results of an inhibition effect of a lithium isobutyrate-L-proline salt on GSK-3β (Ser⁹) in CHO cells: the inhibition effect is better than that of lithium carbonate and lithium isobutyrate;
FIG. 13 shows experimental results of an increase effect of a lithium isobutyrate-L-proline salt on the expression of β-catenine in CHO cells: the effect is better than that of lithium carbonate;
FIG. 14 shows experimental results of an inhibition effect of a lithium isobutyrate-L-proline salt (LisoPro) on the expression of a tau protein in SH-SY5Y cells: the inhibition effect is better than that of lithium carbonate;
FIG. 15 shows experimental results of an effect of a lithium isobutyrate-L-proline salt on the expression of a Notch protein in SH-SY5Y cells: no inhibition effect is achieved, and the effect is better than that of lithium carbonate;
FIG. 16 shows experimental results of the toxicity of a lithium isobutyrate-L-proline salt on HEK293 cells: the toxicity is significantly lower than that of lithium carbonate;
FIG. 17 shows experimental results of the toxicity of lithium isobutyrate and a lithium isobutyrate-L proline salt on HEK293 cells: the toxicity has no obvious difference;
FIG. 18 shows results of the influence of a lithium isobutyrate-L-proline salt and lithium carbonate on the morphology of HEK293 cells: the influence is significantly better than that of lithium carbonate;
FIG. 19 shows experimental results of pharmacokinetics of a lithium isobutyrate-L-proline salt and lithium carbonate in plasma and brain tissues within 24 hours after oral administration;
FIG. 20 shows results of an inhibition effect of a lithium isobutyrate-L-proline salt on GSK-3β in brain tissues of APP/PS1 mice after oral administration: the effect is better than that of lithium carbonate; and
FIG. 21 shows test results of the oral acute toxicity of a lithium isobutyrate-L-proline salt and lithium carbonate.

### DESCRIPTION OF THE EMBODIMENTS

The following embodiments are described to make the present disclosure fully understood by persons skilled in the art, rather than to limit the present disclosure in any manner. Lithium isobutyrate in the present disclosure can be replaced with one or more of lithium butyrate, lithium lactate, lithium cholesterol and other organic lithium salts, and proline can be replaced with valine, lysine and other natural essential amino acids or artificially synthetic amino acids.

### Example 1: Preparation of a lithium isobutyrate-L-proline salt by a single solvent crystallization method

An appropriate amount of a good solvent (n-butanol) was added to equal chemical equivalents of lithium isobutyrate (500 mg) and L-proline (612 mg), heating reflux was conducted to realize proper dissolution, and the use amount of the solvent was recorded (the added volume of the n-butanol was 55 ml). Stirring was conducted for about 3 hours, and heat filtration was conducted, followed by natural cooling and crystallization. A solid was filtered, and vacuum drying was conducted until a constant weight to obtain 589.3 mg of a white solid (a lithium isobutyrate-L-proline salt obtained by a single solvent crystallization method) with a mass yield of 53.06%. XRD and hydrogen spectrum detection were conducted (with CD₃OD as a deuterated reagent).

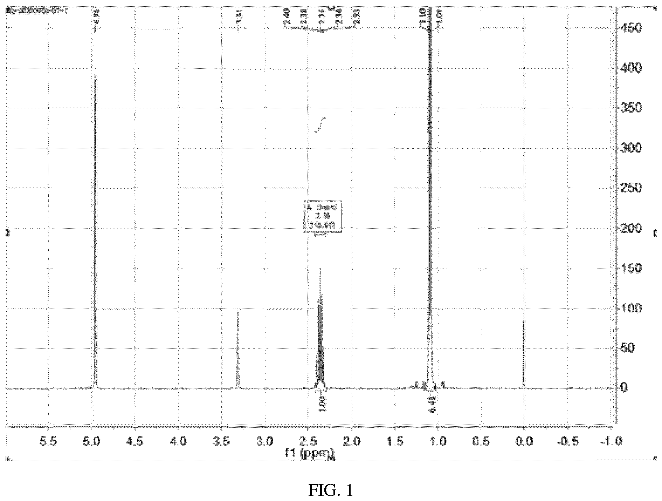

FIG. 1 to FIG. 4 show the hydrogen spectrum of a single compound lithium isobutyrate, the hydrogen spectrum of a single compound L-proline, the hydrogen spectrum of a physical mixture of equal chemical equivalents of lithium isobutyrate and L-proline, and the hydrogen spectrum of a lithium isobutyrate-L-proline salt obtained by a single solvent crystallization method in the present disclosure, respectively.

The hydrogen spectrum of a single compound lithium isobutyrate is (as shown in FIG. 1): ¹H NMR (400 MHz, Methanol-d₄) δ2.36 (hept, J = 6.9Hz, 1H), 1.10 (d, J = 7.0Hz, 6H).

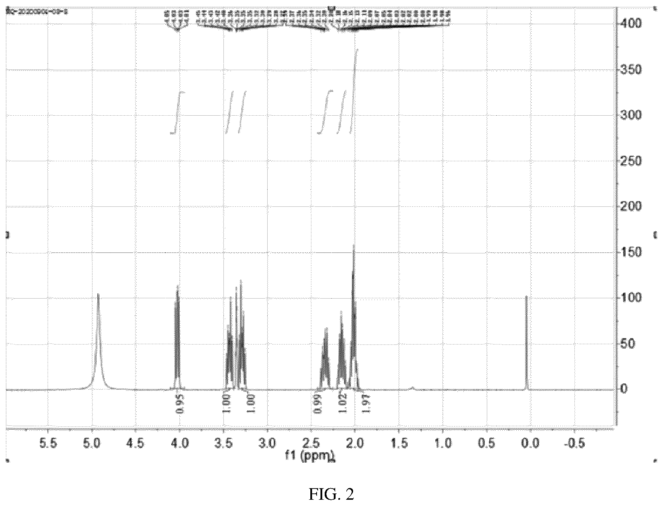

The hydrogen spectrum of a single compound L-proline is (as shown in FIG. 2): ¹H NMR (400 MHz, Methanol-d₄) δ4.03 (dd, J = 8.7, 6.3Hz, 1H), 3.43 (dt, J = 11.5, 6.9Hz, 1H), 3.29 (dt, J = 11.4, 7.3Hz, 1H), 2.42 - 2.27 (m, 1H), 2.16 (td, J = 13.3, 6.4Hz, 1H), 2.07 - 1.96 (m, 2H).

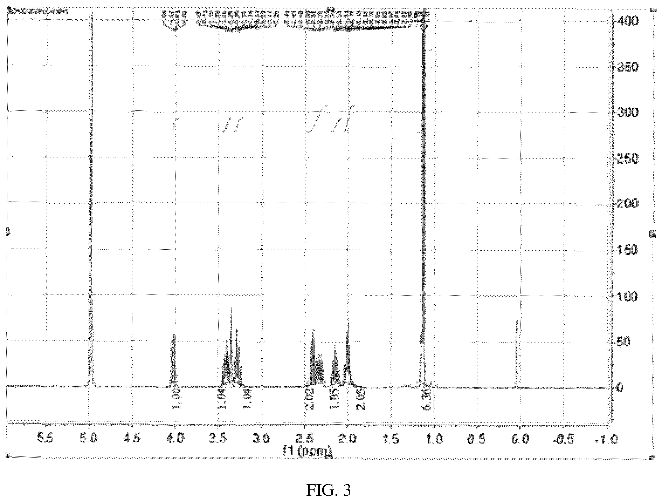

The hydrogen spectrum of a physical mixture of equal chemical equivalents of lithium isobutyrate and L-proline is (as shown in FIG. 3): ¹H NMR (400 MHz, Methanol-d₄) δ4.02 (dd, J = 8.7, 6.1Hz, 1H), 3.41 (dt, J = 11.5, 6.9Hz, 1H), 3.28 (dt, J = 11.4, 7.3Hz, 1H), 2.47 - 2.27 (m, 2H), 2.14 (td, J = 13.3, 6.2Hz, 1H), 2.06 - 1.92 (m, 2H), 1.13 (d, J = 7.0Hz, 6H).

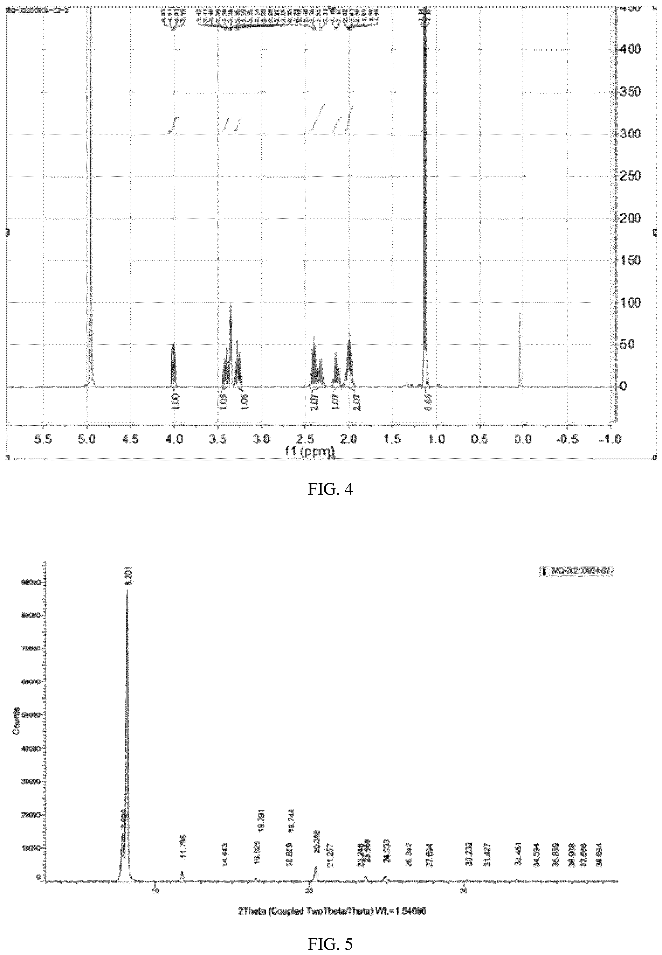

The hydrogen spectrum of a lithium isobutyrate-L-proline salt is (as shown in FIG. 4): ¹H NMR (400 MHz, Methanol-d₄) δ4.01 (dd, J = 8.7, 6.1Hz, 1H), 3.41 (dt, J = 11.5, 6.9Hz, 1H), 3.27 (dt, J = 11.4, 7.3Hz, 1H), 2.47 - 2.26 (m, 2H), 2.14 (dq, J = 13.1, 6.3Hz, 1H), 1.99 (pt, J = 7.3, 4.3Hz, 2H), 1.13 (d, J = 7.0Hz, 6H).

From FIG. 1 to FIG. 4, it can be seen that the chemical equivalent ratio of the lithium isobutyrate to the L-proline in the solid precipitated under the condition of n-butanol in Example 1 is 1:1. In view of a great difference of the solubility of the lithium isobutyrate and the L-proline in the n-butanol, the precipitated compound is not a simple physical mixture, but a salt of the two compounds, and the lithium isobutyrate and the L-proline in the salt are combined at a ratio of 1:1.

FIG. 5 is an XRD pattern of the lithium isobutyrate-L-proline salt obtained in Example 1; and the solid precipitated with the n-butanol as a single solvent has 2θ characteristic peaks at 7.909°, 8.201°, 11.735°, 16.525°, 20.395°, 23.669°, 24.930°, 30.232°, 31.427° and 33.451°.

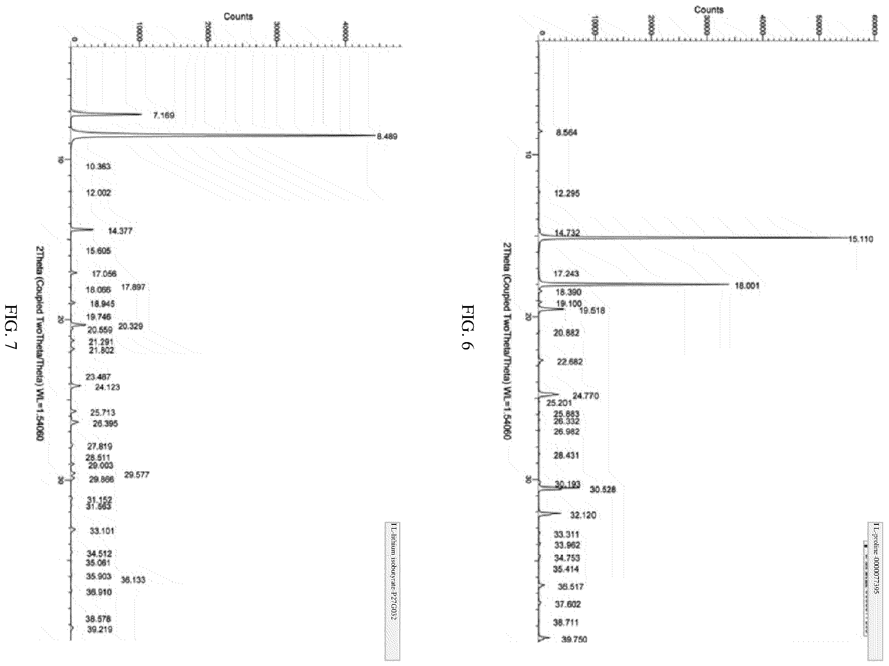

FIG. 6 is an XRD pattern of the single compound L-proline; and the single compound L-proline has 2θ characteristic peaks at 8.564°, 12.295°, 15.110°, 18.001°, 18.390°, 19.100°, 19.518°, 22.682°, 24.770°, 30.528°, 32.120°, 36.517°, 37.602° and 39.750°.

FIG. 7 is an XRD pattern of the single compound lithium isobutyrate; and the single compound lithium isobutyrate has 2θ characteristic peaks at 7.169°, 8.489°, 14.377°, 17.056°, 18.945°, 20.329°, 21.291°, 21.802°, 24.123°, 25.713°, 26.395°, 27.819°, 29.003°, 29.577°, 29.866°, 33 101° and 39.219°.

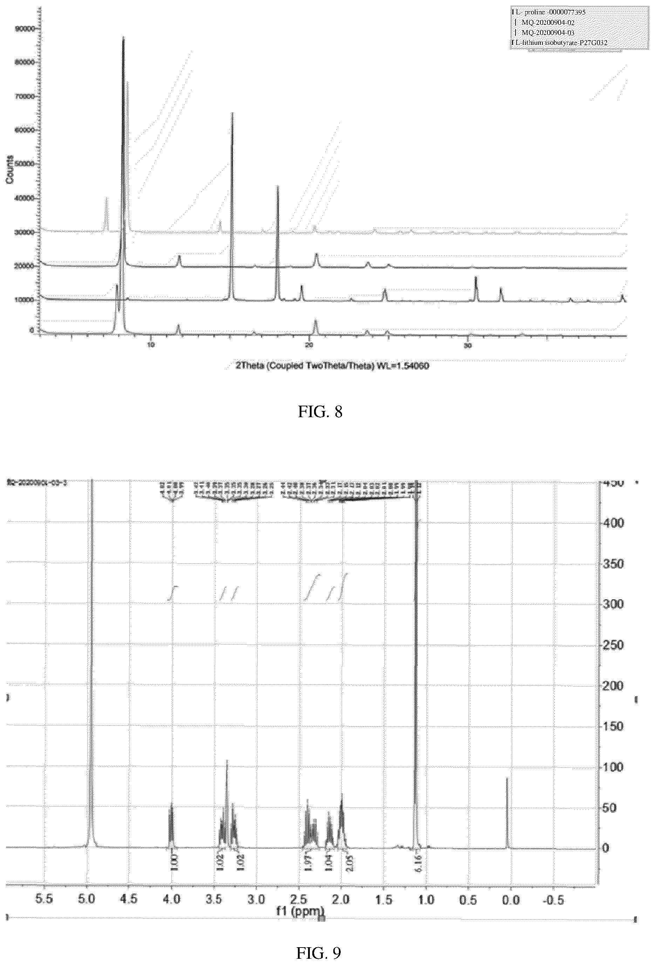

FIG. 8 is a superimposed XRD pattern (from top to bottom: the single compound lithium isobutyrate, a lithium isobutyrate-L-proline salt obtained by using a mixed solvent in Example 2, the single compound L-proline, and the lithium isobutyrate-L-proline salt obtained by using a single solvent in Example 1).

From the superimposed pattern, it can be seen that the hydrogen spectrum of the solid precipitated under the condition of n-butanol in Example 1 is different from that of the single compound lithium isobutyrate, that of the single compound L-proline or the superimposed spectrum of the two single compounds. Therefore, it can be determined that the solid precipitated under the condition of n-butanol is different from the single lithium isobutyrate or the L- proline. Combining with the nuclear magnetic spectrum, it is further proved that the solid obtained in Example 1 of the present disclosure is a lithium isobutyrate-L-proline salt at a ratio of 1:1.

### Example 2: Preparation of a lithium isobutyrate-L-proline salt by a mixed solvent crystallization method

An appropriate amount (50 ml) of a good solvent (ethanol) was added to equal chemical equivalents of lithium isobutyrate (500 mg) and L-proline (612 mg), heating reflux was conducted to realize proper dissolution, and then an appropriate amount (30 ml) of a poor solvent (tetrahydrofuran) was added until proper precipitation of a solid. The good solvent (7 ml) was supplemented to realize redissolution of the solid, and reflux was conducted for 3 hours, followed by natural cooling and crystallization. A solid was filtered, and vacuum drying was conducted until a constant weight to obtain 374.8 mg of a white solid (a lithium isobutyrate-L-proline salt obtained by a mixed solvent crystallization method) with a mass yield of 33.75%. XRD and hydrogen spectrum detection were conducted separately (with CD₃OD as a deuterated reagent).

FIG. 9 shows the hydrogen spectrum of a solid precipitated in a mixed solvent of ethanol and tetrahydrofuran in Example 2.

Combing FIG. 9 with FIG. 1, FIG. 2 and FIG. 3 for analysis, it can be seen that the chemical equivalent ratio of the lithium isobutyrate to the L-proline in the solid precipitated in the mixed solvent of ethanol and tetrahydrofuran in Example 2 is 1:1. In view of a great difference of the solubility of the lithium isobutyrate and the L-proline in the ethanol and the tetrahydrofuran, the precipitated compound is not a simple physical mixture, but a salt, and the lithium isobutyrate and the L-proline in the salt are combined at a ratio of 1:1.

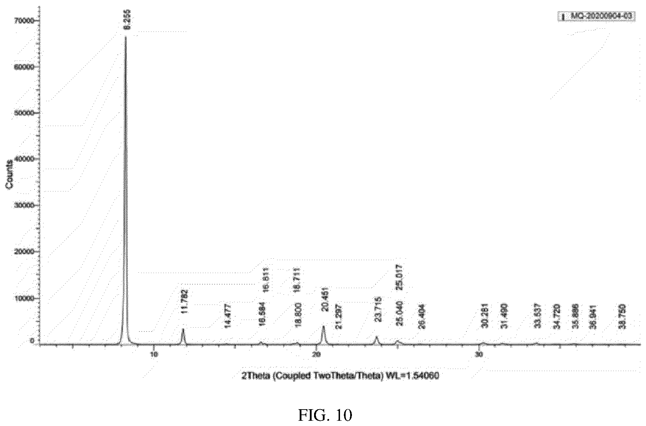

FIG. 10 is an XRD pattern of the solid precipitated in the mixed solvent of ethanol and tetrahydrofuran in Example 2. The salt precipitated in the mixed solvent of ethanol and tetrahydrofuran has 2θ characteristic peaks at 8.255°, 11.782°, 16.584°, 16.811°, 18.711°, 18.800°, 20.451°, 23.715°, 25.017°, 25.040°, 30.281°, 31.490°, 33.537° and 35.886°.

From the superimposed pattern, it can be seen that the hydrogen spectrum of the solid precipitated in the mixed solvent of ethanol and tetrahydrofuran in Example 2 is different from that of the single compound lithium isobutyrate, that of the single compound L-proline or the superimposed spectrum of the two single compounds. Therefore, it can be determined that the solid precipitated in the mixed solvent of ethanol and tetrahydrofuran is a salt different from the two raw materials. From the XRD pattern of the solid precipitated in the mixed solvent of ethanol and tetrahydrofuran and the XRD pattern of the solid precipitated in the single solvent (n-butanol), it can be seen that the two solids have the 2θ characteristic peaks within a deviation range of ±0.2 and same peak intensity characteristics. Therefore, it can be determined that the two solids are a same crystal form of a same salt.

### Example 3: Structural characterization of a salt

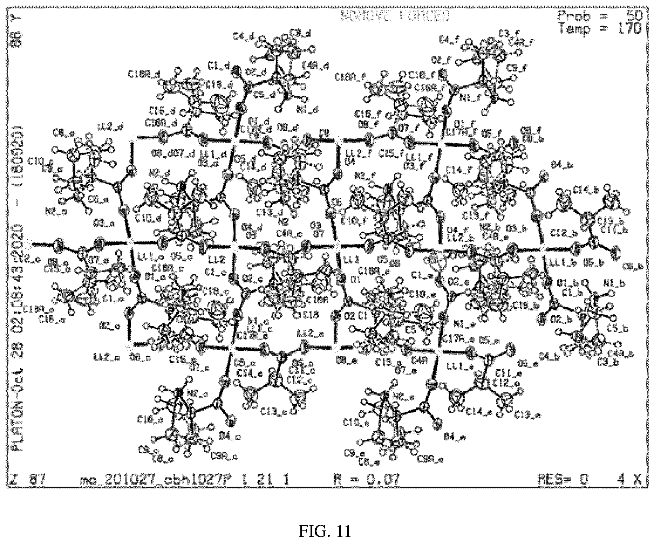

According to the XRD and hydrogen spectrum results, an n-propanol system and an n-butanol system were selected for single crystal culture, and structures of formed solid products were determined. Finally, the mass of a crystal precipitated under the condition of n-butanol meets test requirements, and a single crystal structure is as shown in FIG. 11.

The lengths of C-O bonds of the L-proline in a single crystal lattice were measured by Mercury software, which were 1.233 Å and 1.268 Å respectively, and the ratio was 1.028. The lengths of the C-O bonds of the L-proline are basically the same, indicating that due to obvious proton transfer of the L-proline in the solid product, the lengths of the C-O bonds of a carboxylate group tend to be the same, and it is determined that the prepared product is a lithium isobutyrate-L-proline salt.

### Example 4: Obviously better inhibition effect of a lithium isobutyrate-L-proline salt on GSK-3β in CHO cells than lithium carbonate and lithium isobutyrate

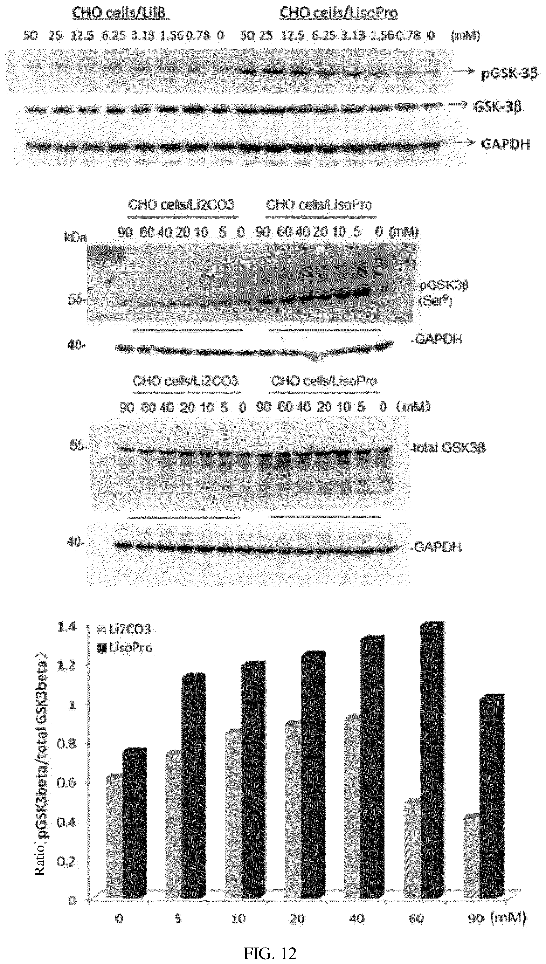

Under conventional culture conditions, CHO cells were treated with lithium isobutyrate or a lithium isobutyrate-L-proline salt with a concentration of 0, 0.78, 1.56, 3.13, 6.25, 12.5, 25 and 50 mM (mmol/L) for 2 hours (or treated with lithium carbonate or a lithium isobutyrate-L-proline salt with a concentration of 0, 5, 10, 20, 40, 60 and 90 mM for 2 hours), and then washed with PBS for three times for 5 minutes each time. The cells were continuously subjected to lysis by an RIPA buffer containing a protease inhibitor, followed by detection by Western blot with phosphorylated GSK-3β (Ser9) or a total GSK-3β antibody as an antibody. GAPDH (glyceraldehyde-3-phosphate dehydrogenase) was used as an internal reference protein. Experimental results are as shown in FIG. 12. The experimental results show that all the lithium isobutyrate-L-proline salt (LisoPro), the lithium carbonate (Li₂CO₃) and the lithium isobutyrate (LiIB) can inhibit the activity of GSK-3β, showing that the expression of the phosphorylated GSK-3β (Ser9) is increased. The lithium isobutyrate-L-proline salt has a dose-dependent inhibition effect on the GSK-3β, and the inhibition effect is stable. Meanwhile, an inhibition effect of the lithium isobutyrate is significantly reduced after the concentration is greater than 12.5 mM, and an inhibition effect of the lithium carbonate on the GSK-3β is obviously lower than that of the lithium isobutyrate-L-proline salt. Therefore, the effect of the lithium isobutyrate-L-proline salt is significantly better than that of the lithium isobutyrate and the lithium carbonate.

The lithium isobutyrate-L-proline salt has a better effect of promoting survival of cells.

### Example 5: Better up-regulation effect of a lithium isobutyrate-L-proline salt on the expression of a cell survival related protein (β-catenine) in CHO cells than lithium carbonate

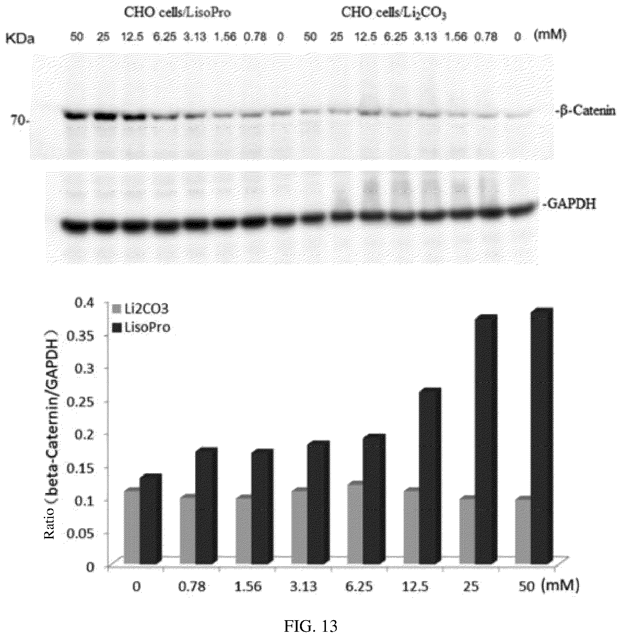

Under conventional culture conditions, CHO cells were treated with lithium carbonate or a lithium isobutyrate-L-proline salt with a concentration of 0, 0.78, 1.56, 3.13, 6.25, 12.5, 25 and 50 mM for 2 hours, and then washed with PBS for three times for 5 minutes each time. The cells were continuously subjected to lysis by an RIPA buffer containing a protease inhibitor, followed by detection by Western blot with a β-catenine antibody as an antibody. GAPDH was used as an internal reference protein. Experimental results are as shown in FIG. 13. The experimental results show that the lithium isobutyrate-L-proline salt significantly increases the expression of a cell survival related protein (β-catenine) in a dose-dependent manner, indicating that the salt has the effect of promoting survival of cells and a significantly better up-regulation effect on the β-catenine than the lithium carbonate.

### Example 6: Better down-regulation effect of a lithium isobutyrate-L-proline salt on the expression of a neurodegeneration related protein (tau) in SH-SY5Y cells than lithium carbonate

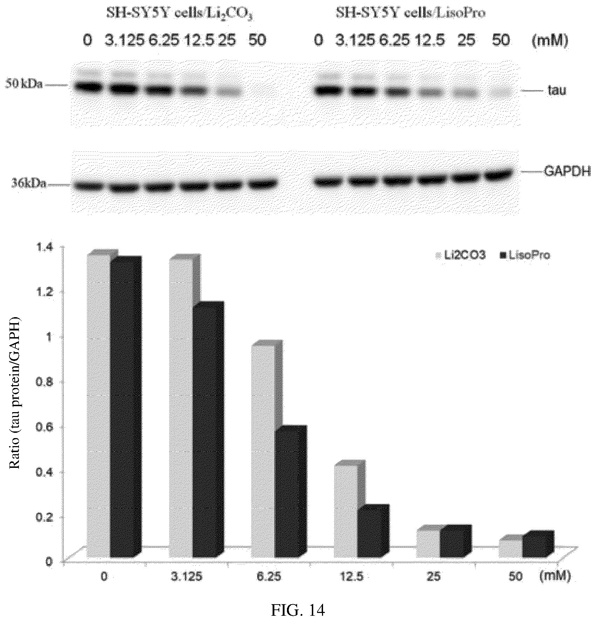

Under conventional culture conditions, SH - SY5Y cells were treated with lithium carbonate or a lithium isobutyrate-L-proline salt with a concentration of 0, 3.125, 6.25, 12.5, 25 and 50 mM for 3 hours, and then washed with PBS for three times for 5 minutes each time. The cells were continuously subjected to lysis by an RIPA buffer containing a protease inhibitor, followed by detection by Western blot with a total tau protein antibody as an antibody. GAPDH was used as an internal reference protein. Experimental results are as shown in FIG. 14. The experimental results show that the lithium isobutyrate-L-proline salt can significantly decrease the expression of a total tau protein in a concentration-dependent manner, and has an effect at a concentration of 3.125 mM. Meanwhile, the lithium carbonate has an effect at a concentration of 6.25 mM, indicating that the lithium isobutyrate-L-proline salt has the effect of inhibiting degeneration of nerve cells, and the effect is significantly better than that of the lithium carbonate.

### Example 7: No influence of a lithium isobutyrate-L-proline salt on the expression of a Notch protein in SH-SY5Y cells

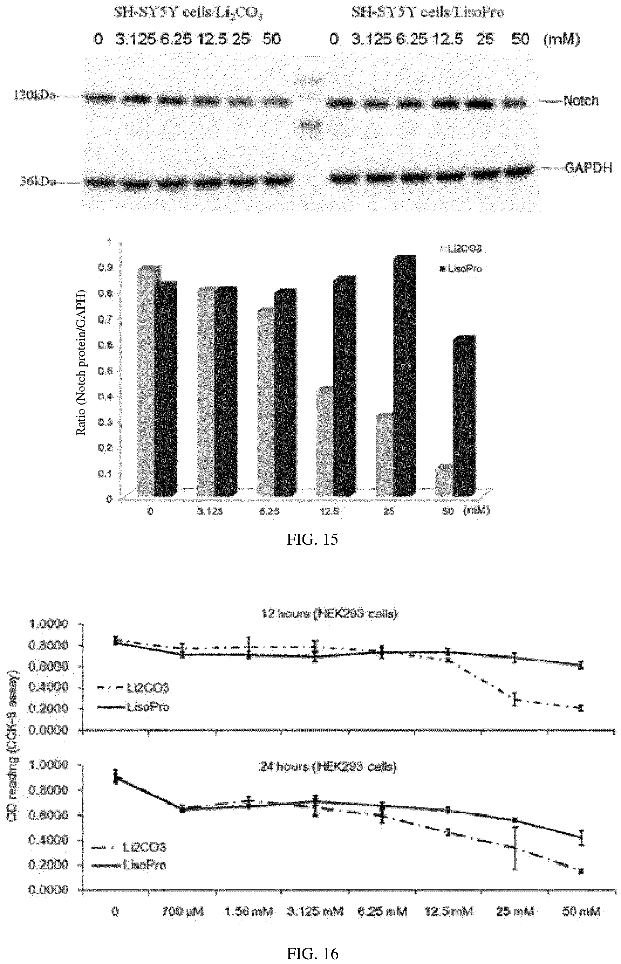

Under conventional culture conditions, SH - SY5Y cells were treated with lithium carbonate or a lithium isobutyrate-L-proline salt with a concentration of 0, 3.125, 6.25, 12.5, 25 and 50 mM for 3 hours, and then washed with PBS for three times for 5 minutes each time. The cells were continuously subjected to lysis by an RIPA buffer containing a protease inhibitor, followed by detection by Western blot with a total Notch protein antibody as an antibody. GAPDH was used as an internal reference protein. A Notch protein is an important signaling molecule at the downstream of GSK-3, and plays an important positive role in regulating the proliferation and survival of cells. Experimental results are as shown in FIG. 15. The experimental results show that the lithium isobutyrate-L-proline salt has no influence on the expression of the Notch at a concentration of less than 50 mM, thus having no influence on the proliferation and survival of cells. Compared with the lithium isobutyrate-L-proline salt, the lithium carbonate as a lithium preparation commonly used in clinical applications has an obvious inhibition effect on the expression of the Notch protein. The above results show that the protective effect of the lithium isobutyrate-L-proline salt on the SH-SY5Y cells is better than that of the lithium carbonate.

The lithium isobutyrate-L-proline salt has lower cytotoxicity than lithium carbonate.

### Example 8: Obviously lower toxicity of a lithium isobutyrate-L-proline salt on HEK293 cells than lithium carbonate

Under conventional culture conditions, HEK293 cells were treated with lithium carbonate (represented by a black dotted line) or a lithium isobutyrate-L-proline salt (represented by a black solid line) with a concentration of 0, 0.7, 1.56, 3.12, 6.25, 12.5, 25 and 50 mM for 12 and 24 hours, and then detected by a CCK-8 test to obtain the cytotoxicity. The absorbance was read by a microplate reader at 450 nm (results are expressed as mean ± standard error). Experimental results are as shown in FIG. 16. The experimental results show that compared with the lithium carbonate, the cytotoxicity of the lithium isobutyrate-L-proline salt is significantly reduced.

### Example 9: No difference in cytotoxicity between a lithium isobutyrate-L-proline salt and lithium isobutyrate

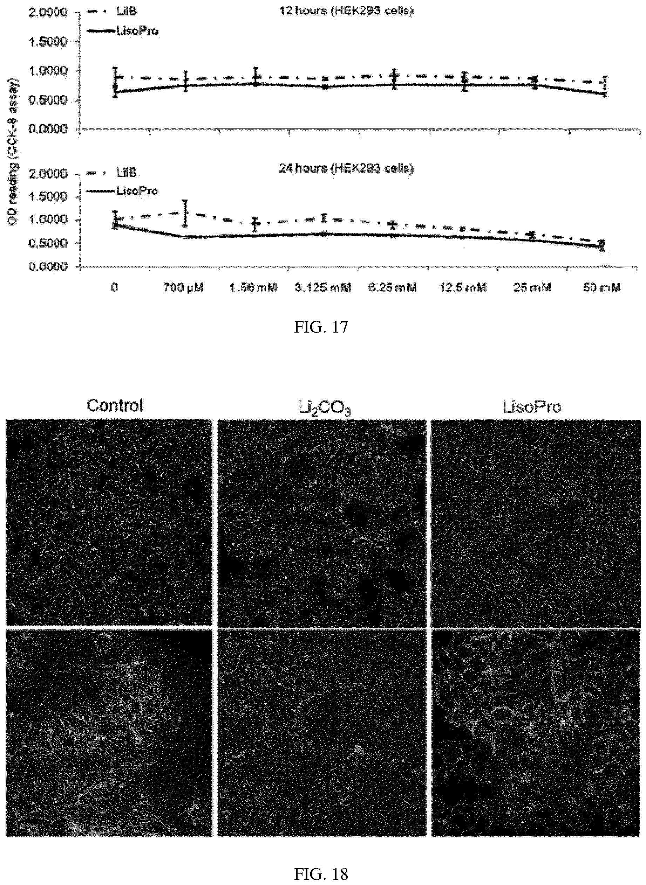

Under conventional culture conditions, HEK293 cells were treated with lithium isobutyrate (represented by a black dotted line) or a lithium isobutyrate-L-proline salt (represented by a black solid line) with a concentration of 0, 0.7, 1.56, 3.12, 6.25, 12.5, 25 and 50 mM for 12 and 24 hours, and then detected by a CCK-8 test to obtain the cytotoxicity. The absorbance was read by a microplate reader at 450 nm (results are expressed as mean ± standard error). Experimental results are as shown in FIG. 17. The experimental results show that compared with the lithium isobutyrate, the lithium isobutyrate-L-proline salt has no obvious difference in the survival of the treated cells.

### Example 10: Influence of a lithium isobutyrate-L-proline salt on the morphology of HEK293 cells

HEK293 cells were separately treated with a lithium isobutyrate-L-proline salt and lithium carbonate with a concentration of 5 mM for 4 hours, then 5 µg/mL of wheat germ agglutinin and an Alexa Fluor 488 conjugate cell membrane dye were added, and the cells were incubated at 37 °C for 10 minutes, washed with PBS for two times and observed under a confocal microscope. Experimental results (as shown in FIG. 18) show that after being treated with the lithium carbonate for 4 hours, the HEK293 cells have an obvious change in morphology, showing that the cells become smaller and are floated. Meanwhile, the cells treated with the lithium isobutyrate-L-proline salt has no obvious change in morphology, indicating that the salt has no obvious cytotoxicity.

Compared with the lithium carbonate, the lithium isobutyrate-L-proline salt has better in vivo pharmacokinetics, inhibitory activity for GSK-3 in brain tissues and safety.

### Example 11: Higher blood lithium level of a lithium isobutyrate-L-proline salt than lithium carbonate

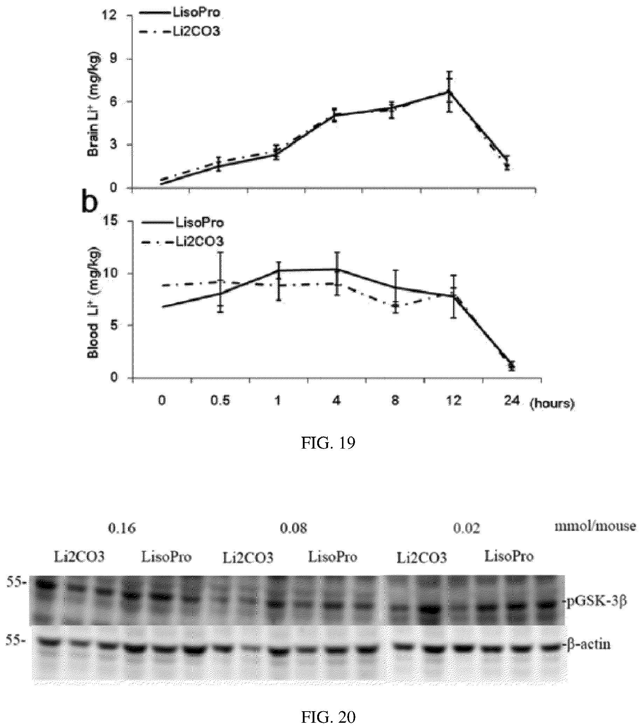

38 male SD rats were administrated with lithium carbonate or a lithium isobutyrate -L proline salt by gavage (at 0.08 mmol lithium/rat, dissolved in 400 µL of double distilled water). After the administration, the rats were sacrificed (1 rat was sacrificed at the time point of 0, and 3 rats were sacrificed at other time points) at different time points (including 0, 0.5, 1, 4, 8, 12 and 24 hours), blood and brain tissues were quickly collected, plasma was separated, and a homogenate of the brain tissues was prepared. The content of lithium in the plasma and the brain tissues was detected by an ICP-MS method (results are expressed as mean ± standard error). Experimental results are as shown in FIG. 19. The experimental results show that compared with the lithium carbonate, the lithium isobutyrate-L-proline salt has a higher blood lithium level, while having no obvious difference in the level of lithium in the brain.

### Example 12: Better influence of a lithium isobutyrate-L-proline salt on the expression of phosphorylated GSK-3β (Ser9) in brain tissues of APP/PS 1 transgenic AD model mice than lithium carbonate after oral administration

APP/PS 1 transgenic AD model mice were orally administrated with a lithium isobutyrate -L proline salt or lithium carbonate (by gavage at 0.02, 0.08 and 0.16 mmol/mouse, with 3 mice in each group), and then sacrificed 4 hours later. Brain tissues were extracted, and a homogenate of the brain tissues was prepared. The expression of phosphorylated GSK-3β (Ser9) in the brain tissues was detected by western blot with a β-actin protein as an internal reference. Experimental results are as shown in FIG. 20. The experimental results show that the effect of the lithium isobutyrate-L-proline salt at dose conditions of 0.02 and 0.08 mmol/mouse is significantly better than that of the lithium carbonate.

### Example 13: Obviously lower oral acute toxicity of a lithium isobutyrate-L-proline salt on mice than lithium carbonate

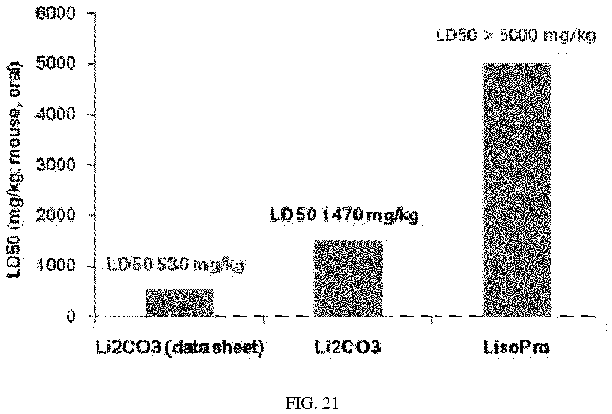

According to preliminary experimental results, the oral acute toxicity of a lithium isobutyrate-L-proline salt and lithium carbonate was detected by a limited test method and a Horn's method, respectively. According to the limited test, 20 Kunming mice (with the weight of 19.6-22 g, including half male mice and half female mice) were orally administrated with 5,000 mg/kg of a lithium isobutyrate-L-proline salt at one time. According to the Horn's method, 40 Kunming mice (with the weight of 19.8-22 g, including half male mice and half female mice) were divided into four groups at an equal ratio of 2.15, including a 464 mg/kg dose group, a 1,000 mg/kg dose group, a 2,150 mg/kg dose group and a 4,640 mg/kg dose group (each group was orally administrated at one time and includes 10 mice with half male mice and half female mice). After the administration, the mice were observed at 0.5, 2 and 4 hours. 24 hours later, poisoning symptoms, occurrence time and death time of the experimental animals were observed every day for an observation period of 14 days. Dead animals and other animals sacrificed within 14 days were subjected to gross anatomy. When gross pathological changes were observed by naked eyes, pathological examination was performed. Experimental results are as shown in FIG. 21. The experimental results show that after the administration with the lithium isobutyrate-L-proline salt, the experimental animals are not dead within 14 days, and no abnormalities are found after the gross anatomy, where the LD50 value is greater than 5,000 mg/kg. After the administration with the lithium carbonate, the experimental animals in the 2,150 mg/kg dose group and the 4,640 mg/kg dose group have abnormal posture symptoms within 24 hours and are all dead, the 464 mg/kg dose group and the 1,000 mg/kg dose group have no abnormalities, and no abnormalities are found after the gross anatomy of the experimental animals in various groups, where the LD50 value is equal to 1,470 mg/kg (according to a report of a document, the LD50 value of the lithium carbonate is 530 mg/kg). The above experimental results show that compared with the lithium carbonate, the oral toxicity of the lithium isobutyrate-L-proline salt is significantly reduced.

## Claims

1. A lithium organic acid-amino acid salt, **characterized in that** a lithium organic acid is one or more of lithium isobutyrate, lithium n-butyrate, lithium lactate, lithium citrate or lithium cholesterol and other lithium organic acids; an amino acid is one of L-proline, valine, lysine or other natural amino acids or artificially synthetic amino acids; and the lithium organic acid-amino acid salt is a salt formed by the lithium organic acid and the amino acid.

2. The lithium organic acid-amino acid salt according to claim 1, **characterized in that** the lithium organic acid is lithium isobutyrate.

3. The lithium organic acid-amino acid salt according to claim 1 or 2, **characterized in that** the amino acid is L-proline.

4. The lithium organic acid-amino acid salt according to claim 3, **characterized in that** the lithium isobutyrate and the amino acid in the lithium organic acid-amino acid salt are combined at a molar ratio of (0.8-1.2):(0.8-1.2).

5. The lithium organic acid-amino acid salt according to claim 3, **characterized in that** the lithium isobutyrate and the L-proline in the lithium organic acid-amino acid salt are combined at a molar ratio of 1:1.

6. A crystal form of a lithium organic acid-amino acid salt, **characterized in that** lithium isobutyrate and L-proline in the lithium organic acid-amino acid salt are combined at a molar ratio of 1:1, and the crystal form has characteristic peaks at a diffraction angle (2θ) of 8.201°±0.2°, 11.735°±0.2°, 20.395°±0.2°, 23.669°±0.2° and 24.930°±0.2° in an X-ray powder diffraction pattern.

7. The crystal form of a lithium organic acid-amino acid salt according to claim 6, **characterized in that** the crystal form has characteristic peaks at a diffraction angle (2θ) of 7.909°±0.2°, 8.201°±0.2°, 11.735°±0.2°, 16.525°±0.2°, 20.395°±0.2°, 23.669°±0.2°, 24.930°±0.2°, 30.232°±0.2°, 31.427°±0.2° and 33.451°±0.2° in an X-ray powder diffraction pattern.

8. The crystal form of a lithium organic acid-amino acid salt according to claim 6, **characterized in that** the crystal form has characteristic peaks at a diffraction angle (2θ) of 7.909°, 8.201°, 11.735°, 16.525°, 20.395°, 23.669°, 24.930°, 30.232°, 31.427° and 33.451° in an X-ray powder diffraction pattern; or the crystal form has characteristic peaks at a diffraction angle (2θ) of 8.255°, 11.782°, 16.584°, 16.811°, 18.711°, 18.800°, 20.451°, 23.715°, 25.017°, 25.040°, 30.281°, 31.490°, 33.537° and 35.886° in an X-ray powder diffraction pattern.

9. A preparation method of the lithium organic acid-amino acid salt according to any one of claims 1-5 or the crystal form according to any one of claims 6-8, **characterized in that** the preparation method comprises: preparation by a single solvent method or a mixed solvent crystallization method.

10. The preparation method according to claim 9, **characterized in that** the preparation method comprises:
in the single solvent method, adding an appropriate amount of a solvent to the lithium organic acid and the amino acid, conducting heating reflux for 1-5 hours, and conducting heat filtration, followed by natural cooling and crystallization;
or in the mixed solvent crystallization method, adding an appropriate amount of a good solvent to the lithium organic acid and the amino acid, conducting heating reflux for dissolution, then adding an appropriate amount of a poor solvent for precipitation of a solid, supplementing the good solvent for redissolution of the solid, and conducting reflux for 1-5 hours, followed by natural cooling and crystallization.

11. The preparation method of the lithium organic acid-amino acid salt according to claim 9 or 10, **characterized in that** in the single solvent method, the solvent is n-butanol; and in the mixed solvent crystallization method, the good solvent is ethanol, and the poor solvent is tetrahydrofuran.

12. A pharmaceutical composition, **characterized in that** the pharmaceutical composition comprises the lithium organic acid-amino acid salt according to any one of claims 1-5 or the crystal form according to any one of claims 6-8.

13. An application of the lithium organic acid-amino acid salt according to any one of claims 1-5, the crystal form according to any one of claims 6-9, or the pharmaceutical composition according to claim12 in preparation of a medicine for treatment of a disease that benefits from the inhibition of the activity of GSK-3β, the increase of the expression of a β-catenine protein, or the inhibition of the expression of a tau protein.

14. The application according to claim 13, **characterized in that** the disease comprises one or more of a neurodegenerative disease and a mental disease.

15. The application according to claim 13, **characterized in that** the neurodegenerative disease is one or more of Alzheimer's disease, Parkinson's disease, Huntington's disease, epilepsy, amyotrophic lateral sclerosis and spinocerebellar ataxia.

16. The application according to claim 13, **characterized in that** the mental disease is one or more of mild, moderate, or severe depression and bipolar disorder.
